# EUROPEAN PATENT APPLICATION

(11) **EP 4 579 203 A1**
(43) Date of publication of application: **02.07.2025**
(21) Application number: 23875229.9
(22) Date of filing: 05.10.2023
(51) Int. Cl.: G01K 7/18, G01K 7/20, G01K 1/024, G01K 1/02, G01K 1/08, G01K 1/14, G01K 3/02, G06F 1/16, A61B 5/01

(54) **TEMPERATURE MEASUREMENT METHOD AND ELECTRONIC DEVICE SUPPORTING SAME**

(30) Priority: 05.10.2022 KR 20220127264; 10.11.2022 KR 20220149654
(71) Applicant: Samsung Electronics Co., Ltd., Suwon-si, Gyeonggi-do 16677 (KR)
(72) Inventor: JUNG, Hyunjun, Suwon-si Gyeonggi-do 16677 (KR); LEE, Jeehoon, Suwon-si Gyeonggi-do 16677 (KR); CHO, Shinhee, Suwon-si Gyeonggi-do 16677 (KR)
(74) Representative: Gulde & Partner
(86) International application number: PCT/KR2023/015311
(87) International publication number: WO 2024/076167

(57) **Abstract**

A wearable device according to an embodiment disclosed in the present document may include a housing having a first surface, a second surface, and a side surface, a display disposed on the first surface, a contact part which is at least partially exposed to the second surface, a temperature sensing element of which a specified parameter value is changed by an object in contact with the contact part, a biometric sensor, a memory, and a processor. The processor may supply a constant current or a constant voltage to the temperature sensing element by using the biometric sensor. The processor may measure the parameter value of the temperature sensing element changed by the constant current or the constant voltage by using the biometric sensor. The processor may determine a temperature of an object in contact with the contact part based on the measured parameter value. In addition to this, an embodiment that may be understood through the specification is possible.

## Description

### TECHNICAL FIELD

Embodiments disclosed in the present document relate to a method of measuring a temperature and an electronic device supporting the same.

### BACKGROUND ART

A wearable device such as a smart watch or a smart ring is being released. The wearable device may perform various health care-related functions such as electrocardiogram measurement, blood pressure measurement, and body temperature measurement.

As methods of measuring a body temperature by an electronic device, 1) an IR sensing method (non-contact method) for measuring radiation-heat, and 2) a method of measuring a temperature of a thermal equilibrium state after thermal conduction are used. The wearable device may be worn to maintain a contact state on the skin of the user, and thus the contact-type temperature sensing may be used.

The contact-type temperature sensing method may use a thermoelectric conversion element. For example, the thermoelectric conversion element may be a resistance temperature detector (RTD) sensor. The RTD sensors have the characteristics of low price and high temperature measurement accuracy.

PT100 is a type of RTD, and uses high-purity platinum (Pt) having the greatest change in characteristics due to temperature. The PT100 enables precise temperature measurement compared to an existing thermistor method, and is widely used in a system for high performance temperature control.

### DISCLOSURE OF THE INVENTION

### TECHNICAL SOLUTION

A wearable device according to an embodiment may include a housing having a first surface, a second surface, and a side surface, a display disposed on the first surface, a contact part which is at least partially exposed to the second surface, a temperature sensing element of which a specified parameter value is changed by an object in contact with the contact part, a biometric sensor, a memory, and a processor. The processor may supply a constant current or a constant voltage to the temperature sensing element by using the biometric sensor. The processor may measure the parameter value of the temperature sensing element changed by the constant current or the constant voltage by using the biometric sensor. The processor may determine a temperature of an object in contact with the contact part based on the measured parameter value.

A temperature measuring method according to an embodiment may be performed by a wearable device including a temperature sensing element. The temperature measurement method may include supplying a constant current or a constant voltage to the temperature sensing element by using a biometric sensor of the wearable device, measuring a parameter value of the temperature sensing element changed by the constant current or the constant voltage by using the biometric sensor, and determining a temperature of an object based on the parameter value.

### BRIEF DESCRIPTION OF THE DRAWINGS

FIG. 1 is a block diagram of an electronic device in a network environment according to various embodiments.
FIG. 2 illustrates a block diagram of a wearable device according to an embodiment.
FIG. 3 is a flowchart illustrating a temperature measurement method according to an embodiment.
FIG. 4 illustrates a wearable device in the form of a smart watch according to an embodiment.
FIG. 5 illustrates driving of a temperature sensing element using an AFE of an electrocardiogram sensor according to an embodiment.
FIG. 6 illustrates a simulation result of the driving the temperature sensing element using the AFE of the electrocardiogram sensor according to an embodiment.
FIG. 7 illustrates driving of a temperature sensing element using a BIA sensor according to an embodiment.
FIG. 8 illustrates driving of a temperature sensing element using a PPG sensor according to an embodiment.
FIG. 9 is an exemplary diagram of temperature measurement and notification display according to an embodiment.
FIG. 10 illustrates a wearable device in the form of a smart ring according to an embodiment.

In relation to the description of drawings, the same or similar reference numerals may be used for the same or similar constituent elements.

### MODE FOR CARRYING OUT THE INVENTION

Hereinafter, various embodiments of the present disclosure will be described with reference to the accompanying drawings. However, this is not intended to limit the present disclosure to the specific embodiments, and it is to be construed to include various modifications, equivalents, and/or alternatives of embodiments of the present disclosure. With regard to the description of the drawings, similar reference numerals may be used to refer to similar elements.

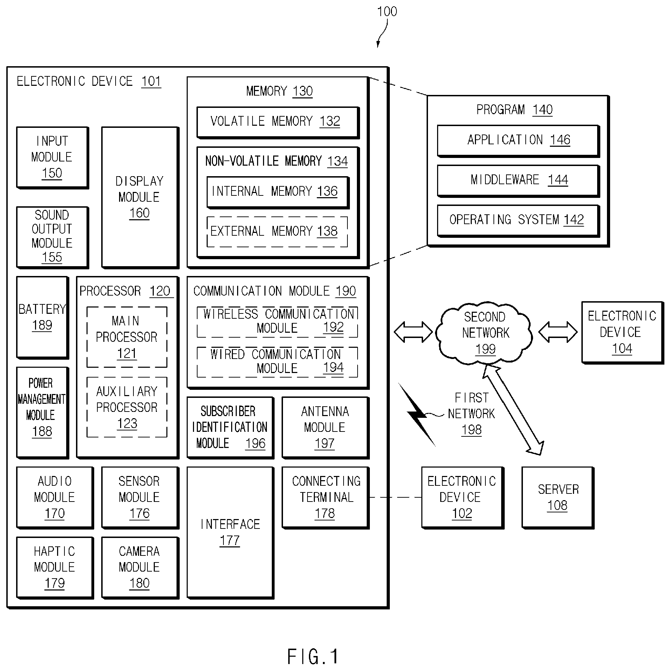

FIG. 1 is a block diagram illustrating an electronic device 101 in a network environment 100 according to various embodiments. Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thererto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a fifth generation (5G) network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a fourth generation (4G) network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the millimeter wave (mmWave) band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

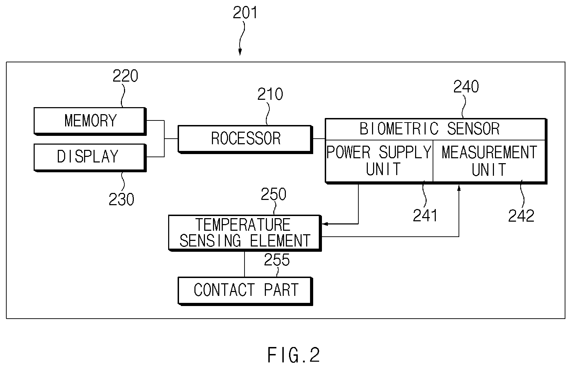

FIG. 2 illustrates a block diagram of a wearable device according to an embodiment.

Referring to FIG. 2, a wearable device 201 (e.g., an electronic device 101 of FIG. 1) may include a processor 210 (e.g., the processor 120 of FIG. 1), a memory 220 (e.g., a memory 130 of FIG. 1), a display 230 (e.g., a display module 160 of FIG. 1), a biometric sensor 240 (e.g., a sensor module 176 of FIG. 1), a temperature sensing element (or a temperature sensor) 250, and a contact part 255.

The processor 210 may perform various operations related to driving of the wearable device 201. According to an embodiment, the processor 210 may measure a temperature of an object in contact with the contact part 255 by using the temperature sensing element 250. The processor 210 may supply power to the temperature sensing element 250 through an analog front end (AFE) of the biometric sensor 240 and measure a changed parameter of the temperature sensing element 250. The processor 210 may determine the temperature based on the measured value of the parameter.

The memory 220 may store various data related to driving of the wearable device 201. According to an embodiment, the memory 220 may store a matching table that associates and stores the characteristics of the temperature sensing element 250 and the temperature. The processor 210 may determine the temperature of the object in contact with the contact part 255 by referring to the changed parameter value of the temperature sensing element 250 and the matching table.

The display 230 may display content such as an image or text. For example, the display 230 may display the temperature of the object measured through the temperature sensing element 250 or may display a notification related to the temperature measurement. According to an embodiment, when the wearable device 201 is a smart ring, the display 230 may be omitted or simplified.

The biometric sensor 240 may acquire biometric information of a user. The biometric sensor 240 may be one of an electrocardiogram (ECG) sensor, a bioelectrical impedance analysis (BIA) sensor, or a photoplethysmography (PPG) sensor. According to an embodiment, the biometric sensor 240 may include a power supply unit 241 and a measurement unit 242. The power supply unit 241 and the measurement unit 242 may constitute an analog front end (AFE) of the biometric sensor 240.

According to an embodiment, the power supply unit 241 may supply power for driving the biometric sensor 240. In addition, the power supply unit 241 may supply power for driving the temperature sensing element 250. For example, the power supply unit 241 may supply a constant current or a constant voltage.

According to an embodiment, the measurement unit (or the measurement circuit unit) 242 may measure data according to driving of the biometric sensor 240. In addition, the measurement unit 242 may be used to measure a change in a parameter related to temperature measurement of the temperature sensing element 250. For example, the measurement unit 242 may be an instrumentation amplifier (IA) or a transimpedance amplifier (TIA).

The temperature sensing element (or the thermoelectric conversion element) 250 may be used to sense the temperature of an object in contact with the contact part 255. The temperature sensing element 250 may receive power from the power supply unit 241 of the biometric sensor 240. For example, the temperature sensing element 250 may receive a current of a specified value from a current source inside the biometric sensor 240. When a resistance value of the temperature sensing element 250 is changed by the current flowing through the temperature sensing element 250, the resistance value of the temperature sensing element 250 may be measured through the measurement unit 242 of the biometric sensor 240.

According to an embodiment, the temperature sensing element 250 may be PT100. A resistance value of PT100 may increase in proportion to a temperature. PT100 may have a temperature-resistance table stored in advance in the memory 220 and may not include a separate calibration operation. The processor 210 may use the temperature-resistance table to determine a temperature that matches the measured resistance value.

The contact part 255 may may come into contact with the skin of the user when the user wears the wearable device 201. For example, in the case of a smart watch, the contact part 255 may be a part of a back cover. For another example, in the case of a smart ring, the contact part 255 may be part of an inner surface of the housing.

The contact part 255 may be implemented with a material having thermal conductivity suitable for measuring a skin temperature. For example, the contact part 255 may be made of glass.

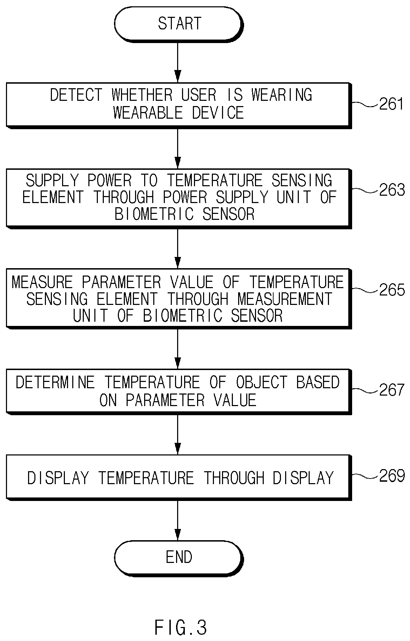

FIG. 3 is a flowchart illustrating a temperature measurement method according to an embodiment.

Referring to FIGS. 2 and 3, in operation 261, the processor 210 may detect whether the user is wearing the wearable device 201. For example, the processor 210 may use a sensor module such as a proximity sensor and a contact sensor to sense whether the user wears the wearable device 201.

In operation 263, the processor 210 may supply power to the temperature sensing element 250 through the power supply unit 241 of the biometric sensor 240. For example, the processor 210 may cause a current of a specified value to flow from a current source inside the biometric sensor 240 to the temperature sensing element 250.

In operation 265, the processor 210 may measure a parameter value (e.g., a resistance value) that changes according to the temperature of the temperature sensing element 250 through the measurement unit 242 inside the biometric sensor 240.

In operation 267, the processor 210 may determine the temperature of the object based on a parameter value (e.g., a resistance value). For example, a temperature-resistance table of the temperature sensing element 1050 may be stored in advance in the memory 220. The processor 210 may use the temperature-resistance table to determine a temperature that matches the measured resistance value.

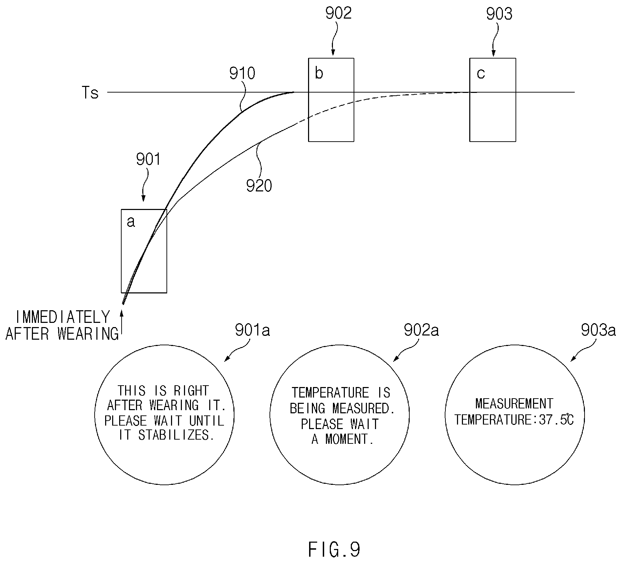

In operation 269, the processor 210 may display the measured temperature on the display 230. The processor 210 may display a notification to the user in a state before the temperature measurement is completed, and may display the measured temperature when the temperature measurement is completed (see FIG. 9).

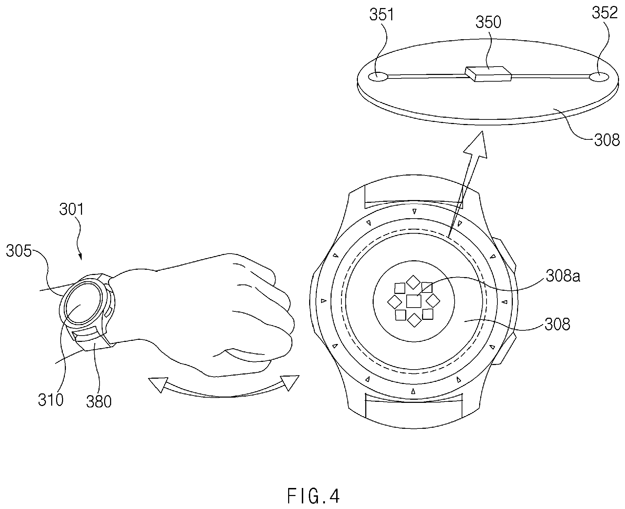

FIG. 4 illustrates a wearable device in the form of a smart watch according to an embodiment. Hereinafter, the discussion will focus on a case where the wearable device is a smart watch, but is not limited thereto.

Referring to FIG. 4, the wearable device 301 (e.g., the electronic device 101 of FIG. 1 or the wearable device 201 of FIG. 2) may be a smart watch. The wearable device 301 may include a housing 305, a display 310, a back cover 308, and a fastening part 380.

The housing 305 may form an appearance of the wearable device 301. The housing 305 may include a first surface (front surface) on which the display 310 is disposed, a second surface (rear surface) coupled to the back cover 308, and a side surface between the first surface and the second surface.

The display 310 may be exposed through the first surface of the housing 305. The display 310 may display an image or text and may receive a user input through a touch sensing circuit.

The back cover (or rear surface plate) 308 may form at least a portion of the second surface of the housing 305. The back cover 308 may be implemented with, for example, coated or colored glass, ceramic, polymer, metal (e.g., aluminum, stainless steel (STS)).

According to an embodiment, the back cover 308 may include a contact part (or a sensor window) 308a (e.g., the contact part 255 of FIG. 2) for temperature sensing. The contact part 308a may be a portion that comes into contact with the wrist when the user wears the wearable device 301. The contact part 308a may be disposed at the center of the back cover 308.

According to an embodiment, a temperature sensing element 350 (e.g., the temperature sensing element 250 of FIG. 2) may be mounted on an inner surface of the back cover 308. For example, the temperature sensing element 350 may be the PT100. When power is supplied to the PT100, the resistance value may vary depending on the temperature.

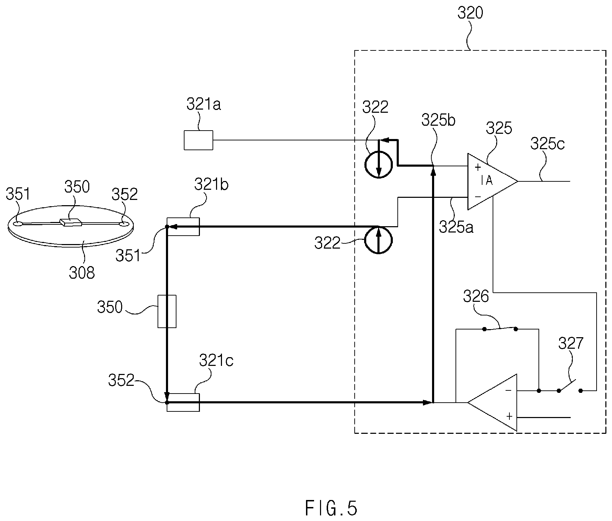

According to an embodiment, the temperature sensing element 350 may receive power through a separate biometric sensor inside the wearable device 301. In addition, a parameter value (e.g., a resistance value) that changes according to the temperature of the temperature sensing element 350 may be measured through a measurement circuit inside the biometric sensor. For example, a first terminal 351 and a second terminal 352 of the temperature sensing element 350 may be connected to a biometric sensor inside the wearable device 301. Additional information related to the connection between the temperature sensing element 350 and the biometric sensor may be provided through FIGS. 5 to 10.

The fastening part 380 may be coupled to the housing 305 to allow the wearable device 301 to be fixed to a part (e.g., a wrist) of the user's body. The fastening part 380 may be implemented with a material such as woven fabric, leather, rubber, urethane, or metal.

FIG. 5 illustrates driving of a temperature sensing element using an AFE of an electrocardiogram sensor according to an embodiment.

Referring to FIGS. 4 and 5, the temperature sensing element 350 may be driven using an analog front end (AFE) of an electrocardiogram (ECG) sensor 320, and a temperature-related parameter value (e.g., a resistance value) may be measured in the temperature sensing element 350.

The electrocardiogram sensor 320 may include first to third electrodes 321a to 321c, an internal current source 322, and a measurement circuit (e.g., an instrumentation amplifier (IA) or a differential amplifier) 325.

The first electrode 321a may be a terminal that contacts a hand different from a hand wearing the wearable device 301. For example, the first electrode 321a may be disposed toward a side surface or a front surface (a surface toward which the display faces) of the housing 305.

The second electrode 321b and the third electrode 321c may be terminals that respectively come into contact with the skin of the user when the user wears the wearable device 201. The first end 351 of the temperature sensing element 350 may be connected to the second electrode 321b, and the second end 352 of the temperature sensing element 350 may be connected to the third electrode 321c.

The internal current source 322 may be mounted to detect the Lead on/off of the electrocardiogram sensor 320. In addition, the internal current source 322 may cause a current of a specified value to flow to the temperature sensing element 350.

The measurement circuit 325 may measure a voltage across both ends of the temperature sensing element 350 and output a resistance value of the temperature sensing element 350. For example, the measurement circuit 325 may be an instrumentation amplifier (IA). A first input terminal 325a of the measurement circuit 325 may be connected to the first end 351 of the temperature sensing element 350, and a second input terminal 325b of the measurement circuit 325 may be connected to the second end 352 of the temperature sensing element 350. An output terminal 325c of the measurement circuit 325 may output a resistance value of the temperature sensing element 350.

When the measurement circuit 325 measures the resistance value of the temperature sensing element 350, switches 326 and 327 inside the electrocardiogram sensor 320 may cause the measurement circuit 325 to operate be separately from the elements inside the surrounding electrocardiogram sensor 320.

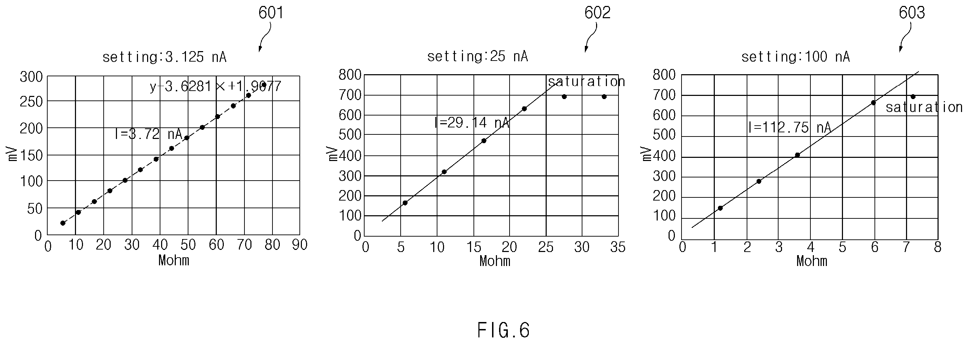

FIG. 6 illustrates a simulation result of driving a temperature sensing element using an AFE of an electrocardiogram sensor according to an embodiment. FIG. 6 is an example and is not limited thereto.

Referring to FIGS. 4 to 6, a current of a specified value may flow to the temperature sensing element 350 through the internal current source 322 of the electrocardiogram sensor 320. The measurement circuit 325 of the electrocardiogram sensor 320 may measure a voltage across both ends of the temperature sensing element 350 to measure the resistance value of the temperature sensing element 350.

A first graph 601 illustrates an output value of the measurement circuit 325 according to a set value and resistance of of the internal current source 322 of the electrocardiogram sensor 320 and, with an actual resistor installed instead of the temperature sensing element 350. In a fixed current source, the resistance and output value may have a linear relationship that is proportional to each other. Through this, it can be confirmed that normal temperature measurement is possible when the temperature sensing element 350 is mounted and power is supplied through the AFE of the electrocardiogram sensor 320 and a resistance value is measured.

A second graph 602 and a third graph 603 are graphs when a current value provided from the current source is changed. Although there are some sections that are saturated, they may have linear characteristics overall, and thus normal temperature measurement may be possible. The temperature sensing element 350 may operate in a non-saturated section and may be used for temperature measurement.

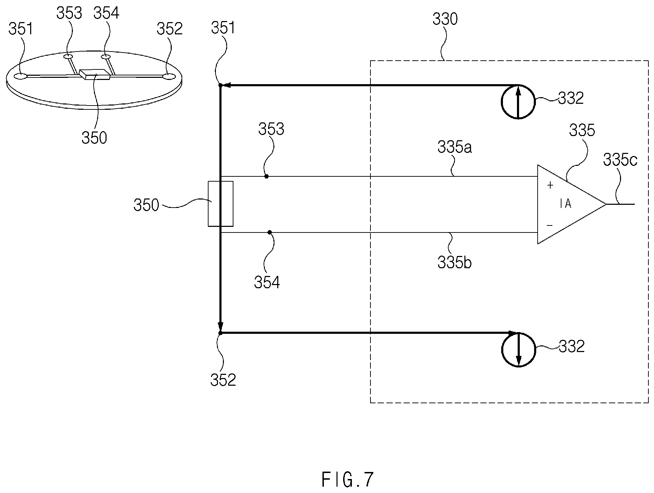

FIG. 7 illustrates driving of a temperature sensing element using a BIA sensor according to an embodiment.

Referring to FIGS. 4 and 7, the temperature sensing element 350 may be driven using an analog front end (AFE) of a bioelectrical impedance analysis (BIA) sensor 330, and a temperature-related parameter value (e.g., a resistance value) may be measured.

The AFE of the bioelectrical impedance analysis (BIA) sensor 330 may include an internal current source 332 and a measurement circuit (e.g., an instrumentation amplifier (IA)) 335.

Each of the first end 351 of the temperature sensing element 350 and the second end 352 of the temperature sensing element 350 may be connected to the internal current source 332. The internal current source 332 may cause a current of a specified value to flow to the temperature sensing element 350.

A third terminal 353 and a fourth terminal 354 of the temperature sensing element 350 may be connected to the measurement circuit 335. The third terminal 353 may be a point between the first terminal 351 and the temperature sensing element 350. The fourth terminal 354 may be a point between the second terminal 352 and the temperature sensing element 350.

The measurement circuit 335 may measure a voltage across both ends of the temperature sensing element 350 and output a resistance value of the temperature sensing element 350. For example, the measurement circuit 335 may be an instrumentation amplifier (IA). A first input end 335a of the measurement circuit 335 may be connected to the third end 353 of the temperature sensing element 350, and a second input end 335b of the measurement circuit 335 may be connected to the fourth end 354 of the temperature sensing element 350. An output terminal 335c of the measurement circuit 335 may output the resistance value of the temperature sensing element 350.

Compared to FIG. 5, when the AFE of the BIA gyro sensor 330 is used, the measurement generated by a resistance component of a conductive wire may be reduced, thereby enabling a relatively accurate temperature measurement.

According to an embodiment, the bioelectrical impedance analysis (BIA) sensor 330 may include a plurality of switches. When the BIA 330 measures bioelectric resistance, the plurality of switches may separate the temperature sensing element 350 from the BIA 330.

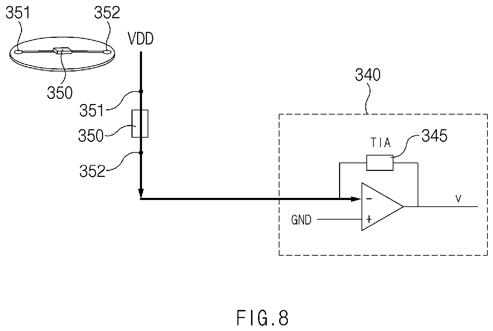

FIG. 8 illustrates driving of a temperature sensing element using a PPG gyro sensor according to an embodiment.

Referring to FIGS. 4 and 8, the temperature sensing element 350 may be driven using an analog front end (AFE) of a photoplethysmography (PPG) sensor 340, and a temperature-related parameter value (e.g., a resistance value) may be measured.

The AFE of the photoplethysmography (PPG) sensor 340 may include a measurement circuit (e.g., a transimpedance amplifier (TIA)) 345.

The first end 351 of the temperature sensing element 350 may be connected to a constant voltage VDD. According to an embodiment, the constant voltage VDD may be provided from the AFE of the PPG sensor 340. The second end 352 of the temperature sensing element 350 may be connected to an input end of the measurement circuit 345.

The measurement circuit 345 may measure a current flowing through the temperature sensing element 350. For example, the measurement circuit 345 may be a transimpedance amplifier (TIA). The processor 210 may calculate the resistance value of the temperature sensing element 350 by causing it to operate in a current measurement mode that keeps the output voltage of the measurement circuit 345 constant.

According to an embodiment, the PPG sensor 340 may include a plurality of switches. When the PPG sensor 340 measures blood flow, the plurality of switches may separate the temperature sensing element 350 from the PPG sensor 340.

FIG. 9 is an exemplary diagram of temperature measurement and notification display according to an embodiment. FIG. 9 is illustrative and is not limited thereto.

Referring to FIGS. 2, 4, and 9, the contact part 355 connected to the temperature sensing element 350 may be implemented with various materials. For example, when it is implemented with a first material (e.g., a Cu element) having a large thermal conductivity coefficient, the time required to reach a safety state temperature Ts may be relatively fast (first graph 910). On the other hand, when it is implemented with a second material (e.g., glass) having a lower thermal conductivity than the first material, the time required to reach the safety state temperature Ts may be relatively long (second graph 920).

The processor 120 may output various notifications until the user wears the wearable device 201 and it becomes a state where temperature measurement is possible.

For example, in an initial state 901 of the second graph 920, the processor 210 may display a first notification 901a. The first notification 901a may be a notification that causes the user to wait until a point at which the wearable device is stabilized after wearing the wearable device.

In a temperature rise state 902 of the second graph 920, the processor 210 may display a second notification 902a. The second notification 902a may be a notification that causes the user to wait until the temperature is measured. For example, when the distribution of the resistance values of the temperature measurement device 350 is greater than or equal to a reference value, the processor 210 may determine it as the temperature increase state 902.

In a stabilization state 903 of the second graph 920, the processor 210 may display a third notification 903a. The third notification 903a may be a notification indicating the measured temperature to the user. The third notification 903a may be a notification that the measured temperature is displayed to the user. The stabilization state 903 may be a state in which the distribution of resistance values of the temperature measurement device 350 is less than (or less than equal to) the reference value.

When the temperature measurement is inaccurate in the initial state 901 or the temperature rise state 902, the processor 210 may calculate the temperature value in the stabilization state 903 in which the distribution of the resistance value is less than (or less than equal to) the reference value. In the stabilization state 903, the processor 120 may determine a temperature value based on an average of resistance values for a few seconds or tens of seconds.

According to an embodiment, the processor 120 may calculate the temperature by reflecting the characteristics of a temperature rise curve in the temperature rise state 902 before the stabilization state 903. For example, in the temperature rise state 902, the processor 120 may estimate a temperature of the stabilization state 903 by using the temperature measured at two different time points (e.g., several seconds) and a pre-calculated fitting curve for the temperature rise curve and display the temperature.

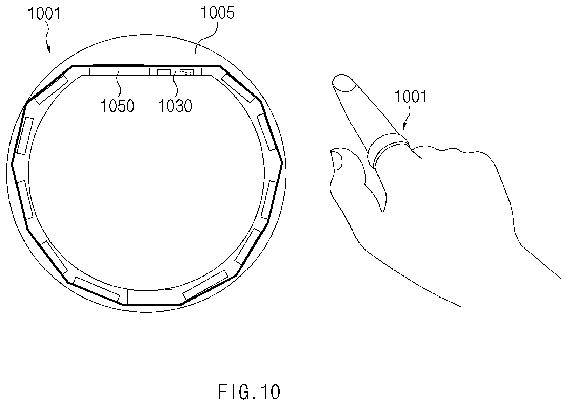

FIG. 10 illustrates a wearable device in the form of a smart ring according to an embodiment.

Referring to FIG. 10, a wearable device 1001 (e.g., the electronic device 101 of FIG. 1 or the wearable device 201 of FIG. 2) may be a smart ring. The wearable device 1001 may include various components such as a processor, a battery, and a wireless communication module inside a housing 1005. In addition, the wearable device 1001 may include a biometric sensor 1030 and a temperature sensor 1050 inside the housing 1005. The biometric sensor 1030 and the temperature sensor 1050 may be disposed adjacent to each other. The biometric sensor 1030 and the temperature sensor 1050 may include terminals or contact parts exposed to an inner surface of the housing 1005 as to come into contact with the user's body. According to an embodiment, when the wearable device 1001 is worn, the contact part may be disposed to correspond to between fingers.

The temperature sensing element 1050 may receive power through the biometric sensor 1030. In addition, a parameter value (e.g., a resistance value) that changes according to the temperature of the temperature sensing element 1050 may be measured through a measurement circuit inside the biometric sensor 1030.

A wearable device according to an embodiment may include a housing including a first surface, a second surface, and a side surface, a display disposed on the first surface, a contact part which is at least partially exposed to the second surface, a temperature sensing element of which a specified parameter value is changed by an object in contact with the contact part, a biometric sensor, a memory, and a processor. The processor may supply a constant current or a constant voltage to the temperature sensing element by using the biometric sensor. The processor may measure the parameter value of the temperature sensing element changed by the constant current or the constant voltage by using the biometric sensor. The processor may determine a temperature of an object in contact with the contact part based on the parameter value.

According to an embodiment, the biometric sensor may be an electrocardiogram (ECG) sensor or a bioelectrical impedance analysis (BIA) sensor.

According to an embodiment, the biometric sensor may include a power supply unit and a measurement unit. The power supply unit may supply the constant current to the temperature sensing element. The measurement unit may measure a resistance value of the temperature sensing element. The processor may determine the resistance value as the parameter value.

According to an embodiment, a first end and a second end of the temperature sensing element may be connected to the power supply unit.

According to an embodiment, a third end and a fourth end of the temperature sensing element may be connected to the measurement unit. The third end may be between the temperature sensing element and the first end, and the fourth end may be between the temperature sensing element and the second end.

According to an embodiment, the measurement unit may be implemented with an instrumentation amplifier (IA) or a differential amplifier.

According to an embodiment, the biometric sensor may be a photoplethysmography (PPG) sensor.

According to an embodiment, the biometric sensor may include a power supply unit and a measurement unit. The power supply unit may supply the constant voltage to the temperature sensing element. The measurement unit may measure a resistance value of the temperature sensing element. The processor may determine the resistance value as the parameter value.

According to an embodiment, the first end of the temperature sensing element may be connected to the power supply unit, and the second end thereof may be connected to the measurement unit.

According to an embodiment, the measurement unit may be implemented with a transimpedance amplifier (TIA).

According to an embodiment, the wearable device may further include a sensor module. The processor may be configured to detect whether a user wears the wearable device through the sensor module, and drive the temperature sensing element when the user is wearing the wearable device.

According to an embodiment, the temperature sensing element may be a thermoelectric conversion element.

According to an embodiment, the memory may store a matching table for the parameter value and a temperature value. The processor may determine the temperature by referring to the matching table.

According to an embodiment, when the distribution of the parameter values is less than or equal to a specified reference value, the processor may determine the temperature by averaging parameter values acquired a plurality of times for a specified time.

According to an embodiment, the wearable device may further include a display. When the distribution of the parameter values is less than or equal to a specified reference value, the processor may display a specified notification through the display.

According to an embodiment, the contact part may be implemented with a material having a specified thermal conduction coefficient or higher.

According to an embodiment, the contact part may be made of glass.

A temperature measuring method according to an embodiment may be performed by a wearable device including a temperature sensing element. The temperature measuring method according to an embodiment may be performed by a wearable device including a temperature sensing element. The temperature measurement method may include an operation of supplying a constant current or a constant voltage to the temperature sensing element by using a biometric sensor of the wearable device, an operation of measuring a parameter value of the temperature sensing element changed by the constant current or the constant voltage by using the biometric sensor, and an operation of determining a temperature of an object based on the parameter value.

According to an embodiment, the biometric sensor may be one of an electrocardiogram (ECG) sensor, a bioelectrical impedance analysis (BIA) sensor, or a photoplethysmography (PPG) sensor.

According to an embodiment, the operation of determining the temperature of the object may include an operation of determining the temperature by referring to a matching table stored in advance in a memory of the wearable device.

A temperature measurement circuit using a thermoelectric conversion element (e.g., PT 100) may be configured with an impedance measurement circuit configured to apply a current and measure a voltage. In order to implement a temperature sensor using a thermoelectric conversion element, it may be necessary to include a circuit that supplies power and a circuit or component that measures resistance. Since the wearable device lacks an internal mounting space, it is necessary to reduce a space for implementing the temperature sensor.

The wearable device according to embodiments disclosed in the present document may operate the temperature sensing element by using an analog front end (AFE) of the biometric sensor. In addition, the electronic device may measure a temperature-related parameter value of the temperature sensing element by using an analog front end (AFE) of the biometric sensor. Through this, an internal mounting space of the wearable device may be secured more widely.

The wearable device according to embodiments disclosed in the present document may not perform a separate calibration process by using a temperature sensing element having a clear relationship between a resistance value and a temperature value.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable device comprising:
a housing having a first surface, a second surface, and a side surface;
a display disposed on the first surface;
a contact part at least partially exposed to the second surface;
a temperature sensing element in which a parameter value designated by an object contacted through the contact part is changed;
a biometric sensor;
a memory; and
a processor controlling the temperature sensing element,
wherein the processor
supplies a constant current or a constant voltage to the temperature sensing element by using the biometric sensor,
measures the parameter value of the temperature sensing element changed by the constant current or the constant voltage by using the biometric sensor, and
determines a temperature of an object in contact with the contact part based on the measured parameter value.

2. The wearable device of claim 1, wherein the biometric sensor is an electrocardiogram (ECG) sensor or a bioelectrical impedance analysis (BIA) sensor.

3. The wearable device of claim 2, wherein the biometric sensor includes a power supply unit and a measurement unit,
the power supply unit supplies the constant current to the temperature sensing element,
the measurement unit measures a resistance value of the temperature sensing element, and
the processor determines the resistance value as the parameter value.

4. The wearable device of claim 3, wherein a first end and a second end of the temperature sensing element are connected to the power supply unit.

5. The wearable device of claim 4, wherein a third end and a fourth end of the temperature sensing element are connected to the measurement unit,
the third end is between the temperature sensing element and the first end, and
the fourth end is between the temperature sensing element and the second end.

6. The wearable device of claim 3, wherein the measurement unit is implemented with an instrumentation amplifier (IA) or a differential amplifier.

7. The wearable device of claim 1, wherein the biometric sensor is a photoplethysmography (PPG) sensor.

8. The wearable device of claim 7, wherein the biometric sensor includes a power supply unit and a measurement unit,
the power supply unit supplies the constant voltage to the temperature sensing element,
the measurement unit measures a resistance value of the temperature sensing element, and
the processor determines the resistance value as the parameter value.

9. The wearable device of claim 3, wherein a first end of the temperature sensing element is connected to the power supply unit, and
a second end thereof is connected to the measurement unit.

10. The wearable device of claim 8, wherein the measurement unit is implemented with an instrumentation amplifier (IA).

11. The wearable device of claim 1, further comprising:
a sensor module,
wherein the processor
senses whether a user is wearing the wearable device through the sensor module, and
drives the temperature sensing element when the user is wearing the wearable device.

12. The wearable device of claim 1, wherein the temperature sensing element is a thermoelectric conversion element.

13. The wearable device of claim 1, wherein the memory stores a matching table for the parameter value and a temperature value, and
the processor determines the temperature by referring to the matching table.

14. The wearable device of claim 1, wherein the processor , when the distribution of the parameter value is less than or equal to a designated reference value, determines the temperature by averaging the parameter values acquired a plurality of times for a designated time.

15. A temperature measurement method performed by a wearable device including a temperature sensing element, the method comprising:
supplying a constant current or a constant voltage to the temperature sensing element by using a biometric sensor of the wearable device;
measuring a parameter value of the temperature sensing element changed by the constant current or the constant voltage by using the biometric sensor; and
determining a temperature of an object based on the measured parameter value.
